# NOUVEAU FASCICULE DE BREVET EUROPEEN

(11) **EP 3 723 722 B2**
(45) Date de publication et mention de la décision concernant l'opposition: **12.11.2025**
(45) Mention de la délivrance du brevet: 16.11.2022
(21) Numéro de dépôt: 18833478.3
(22) Date de dépôt: 12.12.2018
(51) Int. Cl.: A61K 8/73

(54) **SYSTÈME ÉPAISSISSANT ET STABILISANT D'ORIGINE NATURELLE CONVENANT NOTAMMENT À LA PRÉPARATION DE PRODUITS COSMÉTIQUES**
VERDICKUNGS- UND STABILISIERUNGSSYSTEM NATÜRLICHEN URSPRUNGS ZUR HERSTELLUNG VON KOSMETISCHEN ZUSAMMENSETZUNGEN
THICKENER AND STABILIZING SYSTEM OF NATURAL ORIGIN FOR THE PREPARATION OF COSMETIC PRODUCTS

(30) Priorité: 13.12.2017 FR 1762056
(43) Date de publication de la demande: 21.10.2020
(73) Titulaire: ROQUETTE FRERES, 62136 Lestrem (FR)
(72) Inventeur: MENTINK, Léon, 59000 LILLE (FR); LHERITIER, Anne-Marie, 95000 CERGY (FR); LAVARDE, Marc, 95000 CERGY (FR); PIOT, Sophie, 75017 PARIS (FR)
(74) Mandataire: Plasseraud IP
(86) Numéro de dépôt international: PCT/FR2018/053236
(87) Numéro de publication internationale: WO 2019/115944

(56) Documents cités:
- EP-A1- 0 689 770
- DE-A1- 19 816 664
- FR-A1- 2 968 551
- FR-A1- 2 989 892
- Wikipedia "Cellulose"
- DATABASE GNPD [online] MINTEL; April 2016 (2016-04-01), FRESENIUS KABI: "Clear Thickener", XP002780834, Database accession no. 3772239
- GONZÁLEZ-BERMÚDEZ CARLOS A ET AL: "Effect of adding different thickening agents on the viscosity properties andin vitromineral availability of infant formula", FOOD CHEMISTRY, vol. 159, 12 March 2014 (2014-03-12), pages 5 - 11, XP028638688, ISSN: 0308-8146, DOI: 10.1016/J.FOODCHEM.2014.02.168

## Description

Les produits cosmétiques sont des produits formulés réalisés à partir de mélanges, d'association ou de mise en forme d'ingrédients multiples. On recherche aujourd'hui pour des questions environnementales et de soutenabilité, de plus en plus à les formuler à partir d'ingrédients naturels au lieu de synthétiques. Les polymères naturels, sous forme de gommes et de résines, longtemps employés comme liants solubles dans l'eau, agents filmogènes mais aussi épaississants, retrouvent de ce fait une certaine jeunesse.

L'eau étant en général un ingrédient très majoritaire dans ce type de produits. Il convient de l'épaissir c'est-à-dire d'augmenter sa viscosité de manière à rendre les produits cosmétiques plus facilement manipulables et applicables par l'utilisateur final, mais aussi à leur conférer texture agréable d'un point de vue sensoriel sur la peau, les ongles ou les cheveux.

Initialement, l'industrie a eu recours massivement à des polymères d'origine naturelle pour cette fonction d'épaississant et de texturation. Pour autant, ces derniers présentaient un certain nombre d'inconvénients, notamment en terme de couleur, d'odeur, de pureté, de constance d'efficacité et de qualité viscosifiantes.

Ces raisons ont conduit à leur substitution par des polymères synthétiques ou semi-synthétiques. On connaît à ce titre les carbomères très employés en cosmétique. La gamme Carbopol^{®} développée par la société LUBRIZOL en est un exemple. On peut citer en particulier le produit Carbopol^{®} Ultrez 21 qui est un copolymère d'acrylate synthétique, créé pour épaissir, stabiliser et apporter des propriétés de mise en suspension à une grande variété de formules cosmétiques.

Néanmoins, l'industrie des cosmétiques doit aujourd'hui faire face à des nouveaux enjeux en termes de sauvegarde de l'environnement, de préservation de nos ressources fossiles, d'empreinte carbone et de sécurité et de protection des consommateurs. De ce point de vue, elle reconsidère ses formulations et retourne autant que possible vers l'emploi de solutions d'origine naturelle pour la formulation de ses produits. FR 2 989 892 A1, par exemple, décrit des systèmes épaississant et stabilisant des produits cosmétiques comprenant au moins un oligosaccharide phosphorylé et au moins un polymère saccharidique différent des oligosaccharides phosphorylés comme les gommes xanthane, les celluloses et leurs mélanges. La science et la technologie progressent dans ce domaine dans le but de proposer des solutions techniques, fiables, performantes, permettant au formulateur d'épaissir efficacement les produits qu'il réalise, avec des ingrédients naturels, hautement respectueux de l'environnement et du consommateur.

A cet égard, la Demanderesse est parvenue à mettre au point un nouveau système épaississant et stabilisant, constitué d'un mélange ternaire d'au moins un amidon prégélatinisé acétylé, d'au moins une gomme xanthane, et d'au moins une hydroxyéthylcellulose. Ce système épaississant est particulièrement bien adaptée aux formulations cosmétiques.

Le système épaississant selon l'invention permet notamment d'épaissir efficacement des solutions aqueuses et de les stabiliser et ce, de manière totalement inattendue et synergique entre ses différents constituants. A ce titre, la viscosité obtenue en présence du mélange est bien supérieure à celle observée pour chacun des constituants pris séparément, ou pour les combinaisons de 2 seulement de ces constituants.

De plus, le système épaississant selon l'invention permet d'obtenir une très grande stabilité de la viscosité, sans évolution notable de texture et sans synérèse et ce, sur des durées de plusieurs mois. Un tel résultat est particulièrement avantageux, et permet d'atteindre le haut niveau de stabilité requis pour les produits cosmétiques dans lesquels ledit système épaississant est amené à être utilisé.

Au regard de la viscosité et plus particulièrement du comportement rhéologique du milieu aqueux dans lequel il est utilisé, le système épaississant selon l'invention ne conduit pas à un phénomène de synérèse, c'est-à-dire à un démixtion d'eau avec création d'un gel baignant dans une phase aqueuse comme cela est d'ordinaire constaté pour les épaississants naturels pris séparément. Ce résultat est non seulement très intéressant d'un point de vue applicatif, mais il est aussi tout à fait surprenant, car obtenu uniquement par la combinaison ternaire mise en oeuvre : les produits, seuls ou utilisés par couple de 2, conduisent au phénomène rédhibitoire de synérèse.

Du point de vue sensoriel, le système épaississant selon l'invention conduit également à des propriétés particulièrement intéressantes : on obtient ainsi des produits opaques, très blancs et de texture onctueuse, ce qui les positionnent comme des candidats idéaux pour épaissir et stabiliser des émulsions comme des crèmes de soin ou de maquillage. De plus, les produits apparaissent très avantageusement non gras, non filants, et particulièrement doux et frais. combinaisons de ceux-ci.

Aussi, la présente invention consiste en un système épaississant constitué :
a) d'au moins un amidon prégélatinisé acétylé,
b) d'au moins une gomme xanthane,
c) et d'au moins une hydroxyéthylcellulose.

Les combinaisons épaississantes ternaires selon la présente invention comprennent comme composant principal au moins un amidon prégélatinisé acétylé. De tels amidons acétylé comprennent en particulier des polymères d'alpha-glucose prégélatinisé d'origine végétale. Particulièrement préférés sont les amidons prégélatinisés dérivés du maïs, de pomme de terre, du blé, du riz, du pois, de l'avoine, de lentilles, de féveroles, de fèves, de haricots, de pois chiches, ou des combinaisons de ceux-ci.

De préférence, cet amidon prégétatinisé est un amidon waxy, c'est-à-dire riche en amylopectine et pauvre en amylose. Il peut s'agir notamment d'un amidon waxy de maïs, de pomme de terre ou de riz.

Les amidons prégélatinisés sont généralement préparés par des techniques thermiques, chimiques ou mécaniques susceptibles d'engendrer un simple gonflement, un éclatement partiel, voire une solubilisation complète des granules d'amidons de façon ä ce qu'ils deviennent solubles dans l'eau selon un procédé dit à froid, c'est-à-dire par dispersion dans l'eau à une température d'eau inférieure à 45°C, mieux inférieure à 35°C et mieux encore voisine de la température ambiante. Ainsi, de préférence, l'amidon prégélatinisé ne présente plus ou quasiment plus de granules présentant une Croix de Malte en lumière polarisée.

Les techniques préférées d'obtention d'un amidon prégélatinisé sont des techniques de cuisson/séchage de suspensions d'amidon en milieu aqueux comme notamment l'atomisation, la cuisson sur tambour ou l'extrusion. L'auto-clavage ou le chauffage indirect sur échangeur de chaleur sont des procédés de cuisson également possibles et tendent ä produire des dispersions colloïdales complexes consistant en granules intacts, fragmentés et gonflés. On trouvera des exemples de procédé de préparation de tels amidons dans les documents US 3,086,890, US 3,607,394 ou encore FR 2 822 471.

Cet amidon prégélatinisé est modifié avant ou après l'application du traitement de cuisson/séchage décrit plus haut. En termes de modification, il s'agit d'un traitement d'acétylation.

Il est à noter toutefois que les techniques thermiques ou mécaniques susceptibles d'engendrer un simple gonflement, un éclatement partiel, voire une solubilisation complète des granules d'amidons de façon à ce qu'ils deviennent solubles dans l'eau selon un procédé dit à froid, ne sont pas nécessaires dès lors que certains traitements de modifications physico-chimiques ou chimique appliqué à l'amidon sont suffisamment poussés. En effet, il est possible d'utiliser en tant qu'amidon prégélatiné acétylé au sens de l'invention, des produits uniquement dextrinifiés, hydrolysés, cationisés , hydroxypropylés ou caboxyméthylés ne présentant plus ou quasiment plus de granules présentant une Croix de Malte en lumière polarisée.

En particulier, on préfère les amidons prégélatinisés acétylés non ioniques et notamment ceux de la gamme commercialisée par la Demanderesse sous la marque PREGEFLO^{®}. Des exemples de tels amidons les plus préférés sont par exemple le PREGEFLO^{®} CH 40.

La gomme xanthane mise en œuvre dans l'invention est un polysaccharide non ionique. Les gommes de xanthane ont généralement un poids moléculaire compris entre 1 000 000 et 50 000 000 Da. Parmi les produits du commerce possibles, on peut citer par exemple le produit Xanthan Gum FNCS-PC de la société: Jungbunzlauer International AG, le produit Keltrol^{®} CG-T de la société CP Kelco , le produit Cosphaderm^{®} X 17 de la société Cosphatec , le produit Kahlgum 6673 FEE - Xanthan Gum de la société KahlWax , le produits Rhodicare^{®} S et Rhodicaree XC de la société Solvay et le produit VANZAN^{®} NF-C de la société Vanderbilt Minerais.

A titre d'hydroxyéthylcellulose, on peut citer en particulier les produits du commerce suivants : NatrosolTM 250 HHR PC de Ashland Specialty Chemical ; Espesante CH de Chennir ; Tylose^{®} H 15 YG4 de SE Tylose et CellosizeTM HEC QP 40 de DowDuPont (Dow).

Bien que les proportions relatives entre les différents constituants du mélange ternaire ne soient pas fondamentales, il est préférable pour que le système épaississant soit le plus performant que l'amidon prégélatinisé acétylé soit majoritaire par rapport à l'ensemble du système épaississant, soit donc, représente plus de 50 % de l'ensemble du système épaississant. Ainsi, le système épaississant selon l'invention sera préférentiellement constitué de :
a) 1 à 12 parts en poids sec d'au moins un amidon prégélatinisé acétylé,
b) 0,01 à 2 part d'au moins une gomme xanthane,
c) et 0,01 à 3 parts d'au moins une hydroxyéthylcellulose.

Plus préférentiellement, il sera constitué de :
a) 5 à 11 parts en poids sec d'au moins un amidon prégélatinisé acétylé,
b) 0,1 à 1 part d'au moins une gomme xanthane,
c) 0,1 à 2 parts et d'au moins une hydroxyéthylcellulose.

Très préférentiellement, il sera constitué de :
a) 6 à 10 parts en poids sec d'au moins un amidon prégélatinisé acétylé,
b) 0,2 à 0,8 part d'au moins une gomme xanthane,
c) 1 à 1,5 parts et d'au moins une hydroxyéthylcellulose.

Est également décrit un procédé de fabrication d'un système épaissi et stable, à travers les étapes de :
a) fournir une solution aqueuse,
b) chauffer l'eau à une température comprise entre 20°C et 80°C, préférentiellement entre 20°C et 50°C
c) introduire dans la solution aqueuse au moins un amidon prégélatinisé, au moins un polysaccharide non ionique et au moins un polysaccharide anionique
d) agiter le milieu jusqu'à obtenir la dispersion des constituants dans l'eau.

L'homme du métier saura adapter la vitesse d'agitation du milieu, notamment en fonction de la quantité des ingrédients à disperser. Mais une vitesse d'agitation comprise entre 1000 et 5000 tours minute apparaît tout à fait acceptable.

Il est bien entendu par ailleurs, que le procédé selon l'invention reprend à son compte toutes les caractéristiques énumérées plus haut relatives au système ternaire fabriqué selon ledit procédé.

Ce système épaissi s'avère très stable et non allergique pour la peau. Il offre de plus l'avantage de présenter de donner une constance de viscosité et de texture, ne dépendant pas du pH ou de la présence d'électrolytes. En d'autres termes, ce système n'est pas affecté par le pH du milieu, ni par la présence de sels mono, di ou trivalents. Ce critère est d'autant plus important que de manière générale, les produits à usage cosmétique et notamment pour application topique sont susceptibles d'être soumis ou exposés à des variations de pH (à titre d'exemple, le pH de la peau est légèrement acide, et varie entre 4 et 6). Disposer d'un produit qui ne présente pas de limite particulière d'usage en matière de pH ou de présence de sels représente donc un très grand avantage technique pour une composition cosmétique.

Enfin, un dernier objet selon l'invention consiste en une composition cosmétique contenant le système épaissi et stable selon l'invention.

En effet, la système épaissi et stable conforme à l'invention permet la réalisation aisée d'émulsions à la fois très stables et très fines, avec des textures modulables et présentant un toucher frais, soyeux et non gras, même pour des teneurs élevées en phase grasse dispersée. Il est ainsi possible d'obtenir des émulsions ayant un bon effet émollient sur la peau ainsi qu'un bon effet hydratant des couches supérieures de l'épiderme.

Ladite composition cosmétique peut notamment être un produit de soin, comme une composition hydratante, antirides, anti-âge, amincissante, antichante, raffermissante, un body balm, un masque de beauté et se présenter sous forme de solutions épaissies, de gels, de laits , de crèmes, de suspensions , d'aérosols ou de mousses. Ladite composition cosmétique peut notamment être un produit de maquillage pour les yeux, comme un mascara, un liner, ou un produit de maquillage du visage comme une poudre, un fond de teint, du visage, ou un produit de maquillage des ongles comme du vernis, ou un produit de maquillage des lèvres comme un rouge à lèvres ou un brillant à lèvre.

Ladite composition cosmétique peut notamment être un produit solaire comme une protection ou un autobronzant.

Ladite composition cosmétique peut notamment être un produit d'hygiène corporelle comme un savon, un dépilatoire, un déodorant.

Ladite composition cosmétique peut notamment être un produit capillaire comme un shampoing, une coloration, une teinture, une permanente, une lotion antichute, une laque, un fixatif.

Ladite composition cosmétique peut notamment être un parfum, une eau de toilette, une eau de parfum.

Les exemples qui suivent permettront de mieux appréhender la présente invention, sans pour autant en limiter la portée.

### Exemples

Dans l'ensemble des exemples, différentes formulations ont été réalisées, avec les produits suivants :
- Pregeflo CH 40 commercialisé par la société Roquette Frères
- Gomme xanthane commercialisée par la société CP Kelco sous le nom Keltrol^{®} CG-T
- Hydroxyéthylecellulose (HEC) commercialisée par la société Ashland Specialty Chemical sous le nom Natrosol^{™} 250 HHR PC

Sauf précision contraire, toutes les formulations ont été réalisées de la manière suivante :
1) Peser et mélanger les différents épaississants
2) Chauffer l'eau jusqu'à 40°C
3) Sous la turbine en agitation à 2000 tours par minute, disperser le mélange d'ingrédients ajoutés peu à peu
4) Laisser sous agitation jusqu'à ce que l'émulsion soit à température ambiante.

Toutes les viscosités sont déterminées à partir d'un un viscosimètre Brookfield DV-II+ Pro

### Exemple 1

Cet exemple illustre l'effet synergique entre les 3 constituants du système, en vue d'épaissir une solution aqueuse. Le tableau 1 suivant fait apparaître le % en poids dans l'eau de chaque constituant, utilisé seul, en mélange binaire, ou en système ternaire. Le système épaississant selon l'invention conduit avantageusement à la viscosité la plus élevée, tout en donnant une la texture la plus agréable sur la peau.

**Tableau 1**

| **Ingrédients** | **Viscosité (mPas)** |
|---|---|
| 2,5% Pregeflo CH 40 | 15 (SP3) |
| 0,7% Gomme de xanthane | 1 330 (SP3) |
| 0,7% Hydroxyethylcellulose | 770 (SP3) |
| 0,7% Gomme de xanthane + 0,7% Hydroxyethylcellulose | 4470 (SP4) |
| 2,5% Pregeflo CH 40 + 0,7% Gomme de xanthane + 0,7% Hydroxyethylcellulose | 11 135 (SP4) |

### Exemple 2

Cet exemple illustre l'effet de synérèse observé, pour des compositions témoins ne contenant pas la totalité des 3 ingrédients qui constituent le système épaississant selon l'invention. Le tableau 2 suivant fait apparaître le % en poids dans l'eau de chaque constituant utilisé, pour les différentes combinaisons réalisées. Dans tous les cas, on a ajouté un autre constituant qui est l'Amidon M-DF 12 S, amidon granulaire et non un amidon prégélatinisé comme défini plus haut, mais qui n'a pas d'influence véritable sur la viscosité.

On constate que tous les systèmes hors invention conduisent à un phénomène de synérèse, très marquée, ce qui constitue un point rédhibitoire pour leur mise en oeuvre dans des formulations cosmétiques.

**Tableau 2**

| **PREGEFLO CH 40** | **Gomme xanthane** | **HEC** | **Amidon MDF 12S** | **Viscosité (mPa.s) 48 heures à T°C amb.** |
|---|---|---|---|---|
| 1,5 | 0 | 0,7 | 1,8 | Synérèse |
| 2,5 | 0 | 0 | 1,5 | Synérèse |
| 1,9 | 0 | 0 | 2,1 | Synérèse |
| 1,5 | 0 | 0,7 | 1,8 | Synérèse |
| 1,9 | 0 | 0,5 | 1,6 | Synérèse |

### Exemple 3

Cet exemple illustre l'évolution de la viscosité observée pour des solutions aqueuses épaissies avec différents systèmes épaississants selon l'invention, après 15 jours, selon que lesdites solutions aqueuses ont été conservées à 4, 40 ou 50°C. Le tableau 3 démontre un remarquable maintien de la viscosité à toutes ces températures : il n'y a pratiquement pas d'influence de la température de conservation.

**Tableau 3**

| **% Pregeflo CH40** | **% Gomme xanthane** | **% Hydroxyethyl cellulose** | **Viscosité (mPa.s)** | | |
|---|---|---|---|---|---|
| | | | **Après 15 jours à 4°C** | **Après 15 jours à 40°C** | **Après 15 jours à 50°C** |
| 2,5 | 0,3 | 0,2 | 7 100 (SP4) | 6 650 (SP4) | 7 550 (SP4) |
| 4 | 0,6 | 0,2 | 12 650 (SP5) | 11 200 (SP5) | 13 000 (SP5) |
| 4 | 0,3 | 0,8 | 31 000 (SP6) | 28 000 (SP6) | 27 700 (SP6) |
| 3,25 | 0,45 | 1 | 35 5 00 (SP6) | 31 000 (SP6) | 34 150 (SP6) |
| 4 | 0,3 | 0,2 | 15 300 (SP6) | 13 400 (SP6) | 14 500 (SP6) |
| 4,5 | 0,45 | 0,5 | 27 000 (SP6) | 21 000 (SP6) | 23 050 (SP6) |
| 2,5 | 0,3 | 0,8 | 23 000 (SP6) | 20 000 (SP6) | 23 300 (SP6) |
| 3,25 | 0,45 | 0,5 | 16 500 (SP6) | 16 500 (SP6) | 17 500 (SP6) |
| 2,5 | 0,6 | 0,8 | 25 000 (SP6) | 25 700 (SP6) | 24 000 (SP6) |
| 2 | 0,45 | 0,5 | 10 700 (SP6) | 9 100 (SP6) | 10 100 (SP6) |
| 4 | 0,6 | 0,8 | 33 000 (SP6) | 33 000 (SP6) | 34 000 (SP6) |
| 2,5 | 0,6 | 0,2 | 10 600 (SP6) | 9 700 (SP6) | 10 500 (SP6) |
| 3,25 | 0,45 | 0 | 7 000 (SP6) | 7 500 (SP6) | 9 300 (SP6) |
| 3,25 | 0,7 | 0,5 | 18 000 (SP6) | 17 500 (SP6) | 19 200 (SP6) |
| 3,25 | 0,2 | 0,5 | 15 400 (SP6) | 15 100 (SP6) | 17 000 (SP6) |

### Exemple 4

Cet exemple illustre l'évolution de différents paramètres sensoriels observés pour des solutions aqueuses épaissies avec différents systèmes épaississants selon l'invention, après 48 heures. Le tableau 4 démontre que l'ensemble des formulations testées permet d'obtenir un paramétrage sensoriel satisfaisant. En particulier, on obtient toujours un produit peu filant avec un descripteur inférieur ou égal à 3 ; s'étalant bien, avec un descripteur d'étalement supérieur ou égal à 5 et le plus souvent supérieur ou égal à 7, peu collant, avec un descripteur correspondant inférieur ou égal à 4 et pénétrant ; avec un descripteur sensoriel correpsondant supérieur ou égal à 6. A l'exception de certaines formulations riches en hydroxyéthylcellulose, on obtient dans l'ensemble des formulations très blanches, avec des descripteurs de blancheur supérieurs ou égaux à 6.

L'invention permet donc d'obtenir des produits satisfaisants d'un point de vue sensoriel.

| **% PREGEFLO CH40** | **% Gomme xanthane** | **%HEC** | **Evaluations sensorielles après 48h** | | | | |
|---|---|---|---|---|---|---|---|
| | | | **Blanc** | **Filant** | **Etalement** | **Collant** | **Pénétrant** |
| 2,5 | 0,3 | 0,2 | 7 | 2 | 8 | 2 | 8 |
| 4 | 0,6 | 0,2 | 7 | 3 | 7 | 0 | 8 |
| 4 | 0,3 | 0,8 | 6 | 3 | 5 | 3 | 6 |
| 3,25 | 0,45 | 1 | 3 | 2 | 5 | 3 | 6 |
| 4 | 0,3 | 0,2 | 9 | 2 | 7 | 2 | 7 |
| 4,5 | 0,45 | 0,5 | 6 | 3 | 5 | 4 | 8 |
| 2,5 | 0,3 | 0,8 | 8 | 2 | 7 | 0 | 6 |
| 3,25 | 0,45 | 0,5 | 8 | 3 | 7 | 0 | 9 |
| 2,5 | 0,6 | 0,8 | 5 | 2 | 5 | 1 | 7 |
| 2 | 0,45 | 0,5 | 8 | 1 | 7 | 2 | 7 |
| 4 | 0,6 | 0,8 | 4 | 3 | 7 | 2 | 8 |
| 2,5 | 0,6 | 0,2 | 9 | 3 | 9 | 3 | 7 |
| 3,25 | 0,45 | 0 | 9 | 3 | 8 | 3 | 7 |
| 3,25 | 0,7 | 0,5 | 8 | 2 | 8 | 3 | 8 |
| 3,25 | 0,2 | 0,5 | 9 | 2 | 8 | 4 | 8 |

## Revendications

1. - Système épaississant constitué :
a) d'au moins un amidon prégélatinisé acétylé,
b) d'au moins une gomme xanthane,
c) et d'au moins une hydroxyéthylcellulose.

2. - Système épaississant selon la revendication 1, **caractérisé en ce que** l'amidon prégélatinisé est issu du maïs, des pommes de terre, du blé, du riz, du pois, de l'avoine, de lentilles, de féveroles, de fèves, de haricots, de pois chiches, ou des combinaisons de ceux-ci, et en particulier issu d'un amidon waxy et notamment un amidon waxy de maïs, de pomme de terre ou de riz.

3. - Système épaississant selon l'une des revendications précédentes, **caractérisé en ce qu'**il est constitué de :
a) 1 à 12 parts en poids sec d'au moins un amidon prégélatinisé acétylé,
b) 0,01 à 2 parts d'au moins une gomme xanthane,
c) et 0,01 à 3 parts d'au moins une hydroxyéthylcellulose.

4. - Système épaississant selon la revendication 3, **caractérisé en ce qu'**il est constitué de :
a) 5 à 11 parts en poids sec d'au moins un amidon prégélatinisé_acétylé,
b) 0,1 à 1 parts d'au moins une gomme xanthane,
c) 0,1 à 2 parts d'au moins une hydroxyéthylcellulose.

5. - Système épaississant selon la revendication 4, **caractérisé en ce qu'**il est constitué de :
a) 6 à 10 parts en poids sec d'au moins un amidon prégélatinisé acétylé,
b) 0,2 à 0,8 parts d'au moins une gomme xanthane,
c) 1 à 1,5 parts d'au moins une hydroxyéthylcellulose.

6. - Composition cosmétique contenant le système épaissi et stable selon une des revendications 1 à 5.

7. - Composition selon la revendication 6, **caractérisée en ce qu'**il s'agit d'un produit de maquillage pour les yeux, d'un produit solaire, d'un produit d'hygiène corporelle, d'un produit capillaire ou d'un parfum.

## Patentansprüche

1. - Verdickungssystem, bestehend aus:
a) mindestens einer acetylierten vorgelatinierten Stärke,
b) mindestens einem Xanthangummi,
c) und mindestens einer Hydroxyethylcellulose.

2. - Verdickungssystem nach Anspruch 1, **dadurch gekennzeichnet, dass** die vorgelatinierte Stärke aus Mais, Kartoffeln, Weizen, Reis, Erbsen, Hafer, Linsen, Ackerbohnen, Saubohnen, Bohnen, Kichererbsen oder Kombinationen davon und insbesondere aus einer Waxy-Stärke, insbesondere einer Waxy-Stärke aus Mais, Kartoffeln oder Reis gewonnen ist.

3. - Verdickungssystem nach einem der vorhergehenden Ansprüche, **dadurch gekennzeichnet, dass** es besteht aus:
a) 1 bis 12 Teilen Trockengewicht von mindestens einer vorgelatinierten, acetylierten Stärke,
b) 0,01 bis 2 Teilen mindestens eines Xanthangummis,
c) und 0,01 bis 3 Teilen mindestens einer Hydroxyethylcellulose.

4. - Verdickungssystem nach Anspruch 3, **dadurch gekennzeichnet, dass** es besteht aus:
a) 5 bis 11 Teilen Trockengewicht von mindestens einer vorgelatinierten, acetylierten Stärke,
b) 0,1 bis 1 Teilen mindestens eines Xanthangummis,
c) 0,1 bis 2 Teilen mindestens einer Hydroxyethylcellulose.

5. - Verdickungssystem nach Anspruch 4, **dadurch gekennzeichnet, dass** es besteht aus:
a) 6 bis 10 Teilen Trockengewicht von mindestens einer vorgelatinierten, acetylierten Stärke,
b) 0,2 bis 0,8 Teilen mindestens eines Xanthangummis,
c) 1 bis 1,5 Teilen mindestens einer Hydroxyethylcellulose.

6. - Kosmetische Zusammensetzung, die das verdickte und stabile System nach einem der Ansprüche 1 bis 5 umfasst.

7. - Zusammensetzung nach Anspruch 6, **dadurch gekennzeichnet, dass** es sich um ein Augen-Make-up-Produkt, ein Sonnenschutzprodukt, ein Körperhygieneprodukt, ein Haarpflegeprodukt oder ein Parfüm handelt.

## Claims

1. Thickening system consisting of:
a) at least one acetylated pregelatinised starch,
b) at least one xanthan gum,
c) and at least one hydroxyethyl cellulose.

2. Thickening system according to claim 1, **characterised in that** the pregelatinised starch is obtained from maize, potatoes, wheat, rice, peas, oat, lentils, faba beans, broad beans, haricot beans or chickpeas, or combinations thereof, and in particular obtained from a waxy starch and notably a waxy maize, potato or rice starch.

3. Thickening system according to one of the preceding claims, **characterised in that** it consists of:
a) 1 to 12 parts by dry weight at least one acetylated pregelatinised starch,
b) 0.01 to 2 parts at least one xanthan gum,
c) and 0.01 to 3 parts at least one hydroxyethyl cellulose.

4. Thickening system according to claim 3, **characterised in that** it consists of:
a) 5 to 11 parts by dry weight at least one acetylated pregelatinised starch,
b) 0.1 to 1 part at least one xanthan gum,
c) 0.1 to 2 parts at least one hydroxyethyl cellulose.

5. Thickening system according to claim 4, **characterised in that** it consists of:
a) 6 to 10 parts by dry weight at least one acetylated pregelatinised starch,
b) 0.2 to 0.8 parts at least one xanthan gum,
c) 1 to 1.5 parts and at least one hydroxyethyl cellulose.

6. Cosmetic composition containing the stable thickened system according to one of claims 1 to 5.

7. Composition according to claim 6, **characterised in that** it is a makeup product for the eyes, a sun product, a body hygiene product, a haircare product or a fragrance.
